Europäisches Patentamt

European Patent Office

Office européen des brevets

(19)

(11) Numéro de publication: **0 513 103 B1**

(12) **FASCICULE DE BREVET EUROPEEN**

(45) Date de publication de fascicule du brevet: **07.12.94**

(51) Int. Cl.⁵: **C07C 49/17**, C07C 45/86, A61K 7/021, A61K 7/42

(21) Numéro de dépôt: **91903305.0**

(22) Date de dépôt: **29.01.91**

(86) Numéro de dépôt internationale :
**PCT/FR91/00053**

(87) Numéro de publication internationale :
**WO 91/12222 (22.08.91 91/19)**

Le dossier contient des informations techniques présentées postérieurement au dépôt de la demande et ne figurant pas dans le présent fascicule.

(54) **PROCEDE DE PROTECTION DE LA DIHYDROXYACETONE, DIHYDROXYACETONE PROTEGEE PAR CE PROCEDE ET PRODUIT COSMETIOUE CONTENANT UNE TELLE DIHYDROXYACETONE PROTEGEE.**

(30) Priorité: **30.01.90 FR 9001070**

(43) Date de publication de la demande:
**19.11.92 Bulletin 92/47**

(45) Mention de la délivrance du brevet:
**07.12.94 Bulletin 94/49**

(84) Etats contractants désignés:
**AT BE CH DE ES FR GB IT LI LU NL**

(56) Documents cités:
**FR-A- 2 085 208**
**US-A- 4 434 154**

**BEILSTEIN: "Handbuch der organischen Chemie", édition 4, Hauptwerk, tome 1, 1918, Impr. Julius Springer, Berlin, DE; voir page 846, alenéa 6**

(73) Titulaire: **DURAND, Muriel**
**28, rue d'Entraigues**
**F-37000 Tours (FR)**

(72) Inventeur: **DURAND, Muriel**
**28, rue d'Entraigues**
**F-37000 Tours (FR)**

(74) Mandataire: **Dawidowicz, Armand**
**Cabinet Dawidowicz,**
**18, Boulevard Pereire**
**F-75017 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

CHEMICAL ABSTRACTS, vol. 72, no. 20, 18 mai 1970, Columbus, Ohio, US; voir page 223, résumé 103635b

CHEMICAL ABSTRACTS, vol. 100, no. 8, 20 février 1984, Columbus, Ohio, US; M.F. ROBIN et al.: "Study on dihydroxy-acetone preservation as a function of formulation", voir page 318, résumé 56724q

WORLD PATENT INDEX LATEST DATABASE, no. d'accession 83-825600, semaine 47, sections D21, E19, 1983, Derwant Publications Ltd, Londres, GB

SEIFEN, ÖLE, FETTE, WACHSE, vol. 104, no. 9, juin 1978, Augsburg, DE; voir page 247, colonne 2, lignes 1-30

## Description

La présente invention concerne la dihydroxyacétone (DHA), en particulier dans ses applications cosmétiques.

La dihydroxyacétone est utilisée depuis de nombreuses années en cosmétologie pour ses propriétés de pigmentation de la peau qui en font un agent de bronzage utilisé dans ce qu'il est convenu d'appeler des crèmes bronzantes.

Une telle utilisation de la dihydroxyacétone se heurte à de nombreuses difficultés du fait de sa solubilité dans l'eau, de sa capacité d'oxydation, de sa vulnérabilité à la température, de l'instabilité de son pH, de sa sensibilité aux agents microbiens et bactériens, etc. Ces difficultés nécessitent une utilisation très rapide du produit contenant de la dihydroxyacétone après sa fabrication, la portion de dihydroxyacétone restant active diminuant très rapidement dans le temps. En outre, le produit doit être stocké dans des conditions optimales de température et d'hygrométrie afin d'éviter la décomposition et/ou la mise en solution aqueuse de la dihydroxyacétone.

Pour faciliter l'utilisation de la dihydroxyacétone dans l'industrie cosmétologique, il est donc nécessaire de pallier au moins certains de ces inconvénients. Les travaux entrepris à ce sujet ont conduit à la présente invention.

A cet effet, l'invention concerne un procédé de protection et de stabilisation de la dihydroxyacétone (DHA), caractérisé en ce qu'on enferme au moins une partie de la dihydroxyacétone sous forme dimère dans une composition étanche à l'eau libérant la dihydroxyacétone lors de l'application.

La composition étanche à l'eau protège la DHA de l'eau et de l'oxydation et constitue une enveloppe assurant une certaine isolation thermique.

Selon une forme de mise en oeuvre préférée du procédé de l'invention, la composition étanche à l'eau est soluble dans un solvant de manière à maintenir la DHA dimère sous forme cristalline.

De préférence, ladite composition étanche à l'eau contient un mélange de polymères insolubles dans l'eau tels que: acétophtalate de cellulose, polychlorure de vinylidène, copolymères styrène-acrylonitrile, copolymères de diméthylaminoéthylmétacrylate et d'autres esters neutres d'acide métacrylique, acétate de cellulose, nitrocellulose, éthylhydroxyéthylcellulose et éthylcellulose.

Avantageusement, la composition étanche à l'eau contient de l'éthylcellulose en concentration de 0,1% à 10%, de préférence d'environ 2%, par rapport au poids de DHA.

Afin de faciliter la libération de la DHA lors de l'application sur la peau, on peut prévoir que la composition étanche à l'eau contient des corps gras à point de fusion réglé tels qu'un mélange de monoglycérides, de diglycérides et de triglycérides d'acides gras saturés ou insaturés d'une longueur de chaîne comprise entre $C_9$ et $C_{22}$, de préférence entre $C_{12}$ et $C_{18}$, et on contrôle le point de fusion et la dureté par l'adjonction d'hydrocarbures dérivés du pétrole, de silicones et dérivés, de cires minérales, végétales ou animales et/ou d'alcools gras, de leurs esters à longues chaînes et d'acides gras saturés ou insaturés, par exemple acide laurique, myristique, palmitique, oléique, ou stéarique, en particulier, acide stéarique à une concentration de 1% à 15%, de préférence d'environ 7%, par rapport au poids de DHA.

Avantageusement, afin de stabiliser le pH dans une zone acide à neutre à l'intérieur et à proximité immédiate de la composition étanche à l'eau, l'invention prévoit que celle-ci contient un acide organique ou l'un de ses sels, tels qu'acides-phénols, acides- alcools, ou un précurseur d'un tel acide sous forme lactone, en particulier, la glucuno-delta-lactone en concentration de 0,01% à 2%, de préférence d'environ 0,15%, par rapport au poids de DHA.

Selon une forme de mise en oeuvre préférée du procédé selon l'invention, afin d'éviter la dégradation bactérienne de la DHA, on prévoit que ladite composition étanche à l'eau contient un bactériostatique et/ou un bactéricide et/ou un antifongique compatibles avec l'utilisation en cosmétologie, tels que les dérivés de l'isothiazolone, les produits générateurs de formaldéhyde, les dérivés organiques et minéraux du mercure, du brome, du chlore, les acides, les alcools, les phénols et leurs dérivés, en particulier des dérivés de l'acide parahydroxybenzoïque, de ses esters, de ses sels ou de sa solution dans le phénoxyéthanol, à une concentration de 0,05% à 0,5%, de préférence d'environ 0,15%, par rapport au poids de DHA.

Selon l'invention, on applique la composition étanche à l'eau en une seule application ou en plusieurs applications contenant chacune au moins certains des composants de ladite composition étanche à l'eau, par une opération de granulation, de pelliculage, d'enrobage par lit fluidisé ou en turbine restant à la surface de la composition étanche à l'eau. Ces procédés permettent d'assurer qu'une moindre partie de la DHA reste sous forme monomère immédiatement utilisable du point de vue cosmétologique.

L'invention concerne également le produit industriel intermédiaire, dans l'industrie cosmétologique, constitué par la DHA protégée et stabilisée par le procédé précédent, ainsi que les produits cosmétologiques contenant ce produit intermédiaire.

3

Exemple

A titre d'exemple, on va décrire ci-après la composition de deux couches de protection appliquées, selon le procédé de l'invention, à 1kg de DHA. Les pourcentages indiqués sont en poids.

## lère couche

```
Ethylcellulose      (agent d'étanchéité)              1,25%
Acide stéarique   ⎧ agents d'étanchéité, de           4,00%
Triglycérides     ⎨ fusion et d'écrasement con-
                  ⎩ trôlés, adoucissant cosmé-
                    tique                              5,00%
Un plastifiant de l'éthylcellulose dissous
dans de l'alcool ou du chlorure de méthylène.
```

| 2ème couche | |
|---|---|
| glucuno-delta-lactone (stabilisant du pH) | 0,10% |
| acide stéarique (comme dans la couche 1) | 2,00% |
| triglycérides (comme dans la couche 1) | 20,50% |
| silice hydrophobe (étanchéité, contrôle point de fusion) | 0,11% |
| mélange d'esters de (antibactérien) l'acide parahydroxybenzoïque | 0,10% |
| dissous dans de l'alcool ou du chlorure de méthylène. | |

La DHA ainsi traitée s'est révélée exempte des défauts de la DHA non protégée, permettant ainsi une application sûre, à grande échelle et stable dans l'industrie cosmétologique.

## Revendications

1. Un procédé de protection et de stabilisation de la dihydroxyacétone (DHA), caractérisé en ce qu'on enferme au moins une partie de la dihydroxyacétone sous forme dimère dans une composition étanche à l'eau libérant la dihydroxyacétone lors de l'application.

2. Un procédé selon la revendication 1, caractérisé en ce que la composition étanche à l'eau est soluble dans un solvant de manière à maintenir la DHA dimère sous forme cristalline.

3. Un procédé selon l'une des revendications 1 et 2, caractérisé en ce que ladite composition étanche à l'eau contient un mélange de polymères insolubles dans l'eau tels que: acétophtalate de cellulose, polychlorure de vinylidène, copolymères styrène-acrylonitrile, copolymères de diméthylaminoéthylmétacrylate et d'autres esters neutres d'acide métacrylique, acétate de cellulose, nitrocellulose, éthylhydroxyéthylcellulose et éthylcellulose.

4. Un procédé selon la revendication 3, caractérisé en ce que la composition étanche à l'eau contient de l'éthylcellulose en concentration de 0,1% à 10%, de préférence d'environ 2%, par rapport au poids de DHA.

5. Un procédé selon l'une des revendications 1 à 4, caractérisé en ce que la composition étanche à l'eau contient des corps gras à point de fusion réglé tels qu'un mélange de monoglycérides, de diglycérides et de triglycérides d'acides gras saturés ou

4

insaturés d'une longueur de chaîne comprise entre $C_9$ et $C_{22}$, de préférence entre $C_{12}$ et $C_{18}$, et on contrôle le point de fusion et la dureté par l'adjonction d'hydrocarbures dérivés du pétrole, de silicones et dérivés, de cires minérales, végétales ou animales et/ou d'alcools gras, de leurs esters à longues chaînes et d'acides gras saturés ou insaturés, par exemple acide laurique, myristique, palmitique, oléique, ou stéarique, en particulier, acide stéarique à une concentration de 1% à 15%, de préférence d'environ 7%, par rapport au poids de DHA.

6. Un procédé selon l'une des revendications 1 à 5,
caractérisé en ce que la composition étanche à l'eau contient un acide organique ou l'un de ses sels, tels qu'acides-phénols, acides-alcools, ou un précurseur d'un tel acide sous forme lactone, en particulier, la glucuno-delta-lactone en concentration de 0,01% à 2%, de préférence d'environ 0,15%, par rapport au poids de DHA.

7. Un procédé selon l'une des revendications 1 à 6,
caractérisé en ce que ladite composition étanche à l'eau contient un bactériostatique et/ou un bactéricide et/ou un antifongique compatibles avec l'utilisation en cosmétologie, Tels que les dérivés de l'isothiazolone, les produits générateurs de formaldéhyde, les dérivés organiques et minéraux du mercure, du brome, du chlore, les acides, les alcools, les phénols et leurs dérivés, en particulier des dérivés de l'acide parahydroxybenzoïque, de ses esters, de ses sels ou de sa solution dans le phénoxyéthanol, à une concentration de 0,05% à 0,5%, de préférence d'environ 0,15%, par rapport au poids de DHA.

8. Un procédé selon l'une des revendications 1 à 7,
caractérisé en ce qu'on applique la composition étanche à l'eau en une seule application ou en plusieurs applications contenant chacune au moins certains des composants de ladite composition étanche à l'eau, par une opération de granulation, de pelliculage, d'enrobage par lit fluidisé ou en turbine restant à la surface de la composition étanche à l'eau.

9. Dihydroxyacétone protégée et stabilisée par le procédé selon l'une des revendications 1 à 8,
caractérisée en ce qu'elle est enfermée au moins en partie sous forme dimère dans une composition étanche à l'eau libérant la dihydroxyacétone lors de l'application, ladite composition étant disposée selon une ou plusieurs couches d'enrobage.

10. Dihydroxyacétone selon la revendication 9,
caractérisée en ce que la composition étanche à l'eau est soluble dans un solvant de manière à maintenir la DHA dimère sous forme cristalline.

11. Dihydroxyacétone selon l'une des revendications 9 et 10,
caractérisée en ce que ladite composition étanche à l'eau contient un mélange de polymères insolubles dans l'eau tels que: acétophtalate de cellulose, polychlorure de vinylidène, copolymères styrène-acrylonitrile, copolymères de diméthylaminoéthylmétacrylate et d'autres esters neutres d'acide métacrylique, acétate de cellulose, nitrocellulose, éthylhydroxyéthylcellulose et éthylcellulose.

12. Dihydroxyacétone selon la revendication 11,
caractérisée en ce que la composition étanche à l'eau contient de l'éthylcellulose en concentration de 0,1% à 10%, de préférence d'environ 2%, par rapport au poids de DHA.

13. Dihydroxyacétone selon l'une des revendications 9 à 12,
caractérisée en ce que la composition étanche à l'eau contient des corps gras à point de fusion réglé tels qu'un mélange de monoglycérides, de diglycérides et de triglycérides d'acides gras saturés ou insaturés d'une longueur de chaîne comprise entre $C_9$ et $C_{22}$, de préférence entre $C_{12}$ et $C_{18}$, et on contrôle le point de fusion et la dureté par l'adjonction d'hydrocarbures dérivés du pétrole, de silicones et dérivés, de cires minérales, végétales ou animales et/ou d'alcools gras, de leurs esters à longues chaînes et d'acides gras saturés ou insaturés, par exemple acide laurique, myristique, palmitique, oléique, ou stéarique, en particulier, acide stéarique à une concentration de 1% à 15%, de préférence d'environ 7%, par rapport au poids de DHA.

**14.** Dihydroxyacétone selon l'une des revendications 9 à 13,
caractérisée en ce ce que la composition étanche à l'eau contient un acide organique ou l'un de ses sels, tels qu'acides-phénols, acides-alcools, ou un précurseur d'un tel acide sous forme lactone, en particulier la glucunodelta-lactone en concentration de 0,01 à 2%, de préférence d'environ 0,15%, par rapport au poids de DHA.

**15.** Dihydroxyacétone selon l'une des revendications 9 à 14,
caractérisée en ce que ladite composition étanche à l'eau contient un bactériostatique et/ou un bactéricide et/ou un antifongique compatibles avec l'utilisation en cosmétologie, tels que les dérivés de l'isothiazolone, les produits générateurs de formaldéhyde, les dérivés organiques et minéraux du mercure, du brome, du chlore, les acides, les alcools, les phénols et leurs dérivés, en particulier des dérivés de l'acide parahydroxybenzoïque, de ses esters, de ses sels ou de sa solution dans le phénoxyéthanol, à une concentration de 0,05% à 0,5%, de préférence d'environ 0,15%, par rapport au poids de DHA.

**16.** Produit cosmétologique,
caractérisé en ce qu'il comprend de la dihydroxyacétone selon l'une des revendications 9 à 15.

**Claims**

**1.** Method for protecting and stabilising dihydroxyacetone (DHA), characterised in that at least some of the dihydroxyacetone is included in dimeric form in a waterproof composition releasing the dihydroxyacetone at the time of application.

**2.** Method according to Claim 1, characterised in that the waterproof composition is soluble in a solvent so as to keep the dimeric DHA in a crystalline form.

**3.** Method according to one of Claims 1 and 2, characterised in that the said waterproof composition contains a mixture of polymers which are insoluble in water, such as cellulose acetophthalate, vinylidene polychloride, styrene/acrylonitrile copolymers, copolymers of dimethylaminoethyl methacrylate and other neutral esters of methacrylic acid, cellulose acetate, nitrocellulose, ethylhydroxyethyl cellulose and ethyl cellulose.

**4.** Method according to Claim 3, characterised in that the waterproof composition contains ethyl cellulose in a concentration of 0.1% to 10%, and preferably around 2%, with respect to the weight of DHA.

**5.** Method according to one of Claims 1 to 4, characterised in that the waterproof composition contains fatty substances with a regulated melting point such as a mixture of monoglycerides, diglycerides and triglycerides of saturated or unsaturated fatty acids with a chain length between $C_9$ and $C_{22}$ and preferably between $C_{12}$ and $C_{18}$, and the melting point and hardness are controlled by the addition of hydrocarbons derived from petroleum, silicones and derivatives, mineral, vegetable or animal waxes and/or fatty alcohols, long-chain esters thereof and saturated or unsaturated fatty acids, for example lauric, myristic, palmitic, oleic or stearic acid, and in particular stearic acid at a concentration of 1% to 15%, and preferably around 7%, with respect to the weight of DHA.

**6.** Method according to one of Claims 1 to 5, characterised in that the waterproof composition contains an organic acid or one of its salts, such as phenol acids, alcohol acids or a precursor of such an acid in lactone form, in particular glucuno-delta-lactone in a concentration of 0.01% to 2%, and preferably around 0.15%, with respect to the weight of DHA.

**7.** Method according to one of Claims 1 to 6, characterised in that the said waterproof composition contains a bacteriostat and/or bactericide and/or anti-fungal agent compatible with use in cosmetology, such as derivatives of isothiozolone, formaldehyde-generating substances, organic and mineral derivatives of mercury, bromium, chlorine, acids, alcohols, phenols and derivatives thereof, in particular derivatives of parahydroxybenzoic acid, of its esters, of its salts or of its solution in phenoxyethanol, at a concentration of 0.05% to 0.5%, and preferably around 0.15%, with respect to the weight of DHA.

8. Method according to one of Claims 1 to 7, characterised in that the waterproof composition is applied in a single application or in several applications each containing at least some of the components of the said waterproof composition, by means of an operation of granulation, lamination, coating by fluidised bed or in a centrifuge remaining on the surface of the waterproof composition.

9. Dihydroxyacetone protected and stabilised by the method according to one of Claims 1 to 8, characterised in that it is included at least partly in dimeric form in a waterproof composition releasing the dihydroxyacetone at the time of application, the said composition being disposed in one or more coating layers.

10. Dihydroxyacetone according to Claim 9, characterised in that the waterproof composition is soluble in a solvent so as to keep the dimeric DHA in crystalline form.

11. Dihydroxyacetone according to one of Claims 9 and 10, characterised in that the said waterproof composition contains a mixture of polymers which are insoluble in water, such as cellulose acetophthalate, vinylidene polychloride, styrene/acrylonitrile copolymers, copolymers of dimethylaminoethyl methacrylate and other neutral esters of methacrylic acid, cellulose acetate, nitrocellulose, ethylhydroxyethyl cellulose and ethyl cellulose.

12. Dihydroxyacetone according to Claim 11, characterised in that the waterproof composition contains ethyl cellulose in a concentration of 0.1% to 10%, and preferably around 2%, with respect to the weight of DHA.

13. Dihydroxyacetone according to one of Claims 9 to 12, characterised in that the waterproof composition contains fatty substances with a regulated melting point such as a mixture of monoglycerides, diglycerides and triglycerides of saturated or unsaturated fatty acids with a chain length between $C_9$ and $C_{22}$ and preferably between $C_{12}$ and $C_{18}$, and the melting point and hardness are controlled by the addition of hydrocarbons derived from petroleum, silicones and derivatives, mineral, vegetable or animal waxes and/or fatty alcohols, long-chain esters thereof and saturated or unsaturated fatty acids, for example lauric, myristic, palmitic, oleic or stearic acid, and in particular stearic acid at a concentration of 1% to 15%, and preferably around 7%, with respect to the weight of DHA.

14. Dihydroxyacetone according to one of Claims 9 to 13, characterised in that the waterproof composition contains an organic acid or one of its salts, such as phenol acids, alcohol acids or a precursor of such an acid in lactone form, in particular glucuno-delta-lactone in a concentration of 0.01% to 2%, and preferably around 0.15%, with respect to the weight of DHA.

15. Dihydroxyacetone according to one of Claims 9 to 14, characterised in that the said waterproof composition contains a bacteriostat and/or bactericide and/or anti-fungal agent compatible with use in cosmetology, such as derivatives of isothiozolone, formaldehyde-generating substances, organic and mineral derivatives of mercury, bromium, chlorine, acids, alcohols, phenols and derivatives thereof, in particular derivatives of parahydroxybenzoic acid, of its esters, of its salts or of its solution in phenoxyethanol, at a concentration of 0.05% to 0.5%, and preferably around 0.15%, with respect to the weight of DHA.

16. Cosmetological product, characterised in that it comprises dihydroxyacetone according to one of Claims 9 to 15.

**Patentansprüche**

1. Verfahren zum Schutz und zur Stabilisierung von Dihydroxyaceton (DHA), **dadurch gekennzeichnet**, daß wenigstens ein Teil des Dihydroxyacetons in dimerer Form in einer wasserdichten Mischung eingeschlossen wird, die das Dihydroxyaceton bei der Anwendung freisetzt.

2. Verfahren nach Anspruch 1, **dadurch gekennzeichnet**, daß die wasserdichte Mischung in einem Lösungsmittel löslich ist, derart, daß das dimere DHA in kristalliner Form aufrechterhalten wird.

3. Verfahren nach einem der Ansprüche 1 und 2, **dadurch gekennzeichnet**, daß die genannte wasserdichte Mischung ein Gemisch von wasserunlöslichen Polymeren enthält, beispielsweise Celluloseacetophtalat, Polyvinylidenchlorid, Styrolacrylonitrilcopolymere, Copolymere des Dimethylaminoethylmetacrylats und anderer neutraler Ester der Metacrylsäure, Celluloseacetat, Nitrocellulose, Ethylhydroxyethylcellulose und Ethylcellulose.

4. Verfahren nach Anspruch 3, **dadurch gekennzeichnet**, daß die wasserdichte Mischung Ethylcellulose mit einer Konzentration von 0,1 % bis 10 %, vorzugsweise ungefähr 2 %, im Verhältnis zum Gewicht des DHA enthält.

5. Verfahren nach einem der Ansprüche 1 bis 4, **dadurch gekennzeichnet**, daß die wasserdichte Mischung Fett mit eingestelltem Schmelzpunkt enthält, beispielsweise eine Mischung von Monoglyceriden, Diglyceriden und Triglyceriden gesättigter oder ungesättigter Fettsäuren einer Kettenlänge zwischen $C_9$ und $C_{22}$, vorzugsweise zwischen $C_{12}$ und $C_{18}$, und daß der Schmelzpunkt und die Härte gesteuert werden durch Hinzufügen von Kohlenwasserstoffen, die Derivate des Erdöls sind, von Siliconen und Derivaten, von Mineralwachsen, pflanzlichen oder tierischen Fetten und/oder Fettalkoholen, von deren langkettigen Estern und von gesättigten oder ungesättigten Fettsäuren, beispielsweise Laurinsäure, Myristinsäure, Palmitinsäure, Ölsäure oder Stearinsäure, insbesondere Stearinsäure mit einer Konzentration von 1 % bis 15 %, vorzugsweise ungefähr 7 %, im Verhältnis zum Gewicht des DHA.

6. Verfahren nach einem der Ansprüche 1 bis 5, **dadurch gekennzeichnet**, daß die wasserdichte Mischung eine organische Säure oder eines derer Salze enthält, beispielsweise Phenolsäuren, Alkoholsäuren oder eine Vorstufe einer solchen Säure in Lactonform, insbesondere das Gluconsäure-deltalacton mit einer Konzentration von 0,01 % bis 2 %, vorzugsweise ungefähr 0,15 %, im Verhältnis zum Gewicht des DHA.

7. Verfahren nach einem der Ansprüche 1 bis 6, **dadurch gekennzeichnet**, daß die genannte wasserdichte Mischung einen bakteriostatischen und/oder einen bakteriziden und/oder einen fungiziden Stoff enthält, die mit der Verwendung in der Kosmetologie verträglich sind, beispielsweise die Derivate des Isothiazolons, die erzeugenden Mittel des Formaldehyds, die organischen Derivate und Minerale des Quecksilbers, des Broms, des Chlors, Säuren, Alkohole, Phenole und deren Derivate, insbesondere Derivate der Parahydroxybenzoesäure, deren Ester, deren Salze oder deren Lösung in Phenoxyethanol, mit einer Konzentration von 0,05 % bis 0,5 %, vorzugsweise ungefähr 0,15 %, im Verhältnis zum Gewicht des DHA.

8. Verfahren nach einem der Ansprüche 1 bis 7, **dadurch gekennzeichnet**, daß man die wasserdichte Mischung in einem einzigen oder in mehreren Aufträgen aufträgt, die jeden oder wenigstens bestimmte der Inhaltsstoffe genannter wasserdichter Mischung enthalten, durch einen Granuliervorgang, einen Filmbildungsvorgang, einen Ummantelungsvorgang in einer Wirbelschicht oder in einer Zentrifuge, wobei an der Oberfläche die wasserdichte Mischung übrigbleibt.

9. Dihydroxyaceton, das geschützt und stabilisiert ist durch das Verfahren nach einem der Ansprüche 1 bis 8, **dadurch gekennzeichnet**, daß es wenigstens teilweise in dimerer Form in einer wasserdichten Mischung eingeschlossen ist, die das Dihydroxyaceton bei der Anwendung freisetzt, wobei genannte Mischung entlang einer oder mehrerer Umhüllungsschichten angeordnet ist.

10. Dihydroxyaceton nach-Anspruch 9, **dadurch gekennzeichnet**, daß die wasserdichte Mischung in einem Lösungsmittel löslich ist, derart, daß das dimere DHA in kristalliner Form aufrechterhalten bleibt.

11. Dihydroxyaceton nach einem der Ansprüche 9 und 10, **dadurch gekennzeichnet**, daß genannte wasserdichte Mischung ein Gemisch von wasserunlöslichen Polymeren enthält, beispielsweise Celluloseacetophtalat, Polyvinylidenchlorid, Styrolacrylonitrilcopolymere, Copolymere des Dimethylaminoethylmetacrylats und anderer neutraler Ester der Metacrylsäure, Celluloseacetat, Nitrocellulose, Ethylhydroxyethylcellulose und Ethylcellulose.

12. Dihydroxyaceton nach Anspruch 11, **dadurch gekennzeichnet**, daß die wasserdichte Mischung Ethylcellulose mit einer Konzentration von 0,1 % bis 10 %, vorzugsweise ungefähr 2 %, im Verhältnis zum Gewicht des DHA enthält.

13. Dihydroxyaceton nach einem der Ansprüche 9 bis 12, **dadurch gekennzeichnet**, daß die wasserdichte Mischung Fett mit eingestelltem Schmelzpunkt enthält, beispielsweise eine Mischung von Monoglyceriden, Diglyceriden und Triglyceriden gesättigter oder ungesättigter Fettsäuren einer Kettenlänge zwischen $C_9$ und $C_{22}$, vorzugsweise zwischen $C_{12}$ und $C_{18}$, und daß der Schmelzpunkt und die Härte gesteuert werden durch Hinzufügen von Kohlenwasserstoffen, die Derivate des Erdöls sind, von Siliconen und Derivaten, von Mineralwachsen, pflanzlichen oder tierischen Fetten und/oder Fettalkoholen, von deren langkettigen Estern und von gesättigten oder ungesättigten Fettsäuren, beispielsweise Laurinsäure, Myristinsäure, Palmitinsäure, Ölsäure oder Stearinsäure, insbesondere Stearinsäure mit einer Konzentration von 1 % bis 15 %, vorzugsweise ungefähr 7 %, im Verhältnis zum Gewicht des DHA.

14. Dihydroxyaceton nach einem der Ansprüche 9 bis 13, **dadurch gekennzeichnet**, daß die wasserdichte Mischung eine organische Säure oder eines ihrer Salze enthält, beispielsweise Phenolsäuren, Alkoholsäuren oder eine Vorstufe einer solchen Säure in Lactonform, insbesondere das Gluconsäure-delta-lacton mit einer Konzentration von 0,01 bis 2 %, vorzugsweise ungefähr 0,15 %, im Verhältnis zum Gewicht des DHA enthält.

15. Dihydroxyaceton nach einem der Ansprüche 9 bis 14, **dadurch gekennzeichnet**, daß genannte wasserdichte Mischung einen bakteriostatischen und/oder einen bakteriziden und/oder einen fungiziden Stoff enthält, die mit der Verwendung in der Kosmetologie verträglich sind, beispielsweise die Derivate des Isothiazolons, die erzeugenden Mittel des Formaldehyds, die organischen Derivate und Minerale des Quecksilbers, des Broms, des Chlors, Säuren, Alkohole, Phenole und deren Derivate, insbesondere die Derivate der Parahydroxybenzoesäure, deren Ester, deren Salze oder deren Lösung in Phenoxyethanol, mit einer Konzentration von 0,05 % bis 5 %, vorzugsweise ungefähr 0,15 %, im Verhältnis zum Gewicht des DHA.

16. Kosmetisches Produkt, **dadurch gekennzeichnet**, daß es das Dihydroxyaceton nach einem der Ansprüche 9 bis 15 enthält.